# EUROPÄISCHE PATENTSCHRIFT

(11) **EP 0 570 829 B1**
(45) Veröffentlichungstag und Bekanntmachung des Hinweises auf die Patenterteilung: **25.04.2001**
(21) Anmeldenummer: 93107728.3
(22) Anmeldetag: 12.05.1993
(51) Int. Cl.: A61K 38/08, A61K 31/19, A61K 9/107

(54) **Cyclosporin(e) enthaltende pharmazeutische Zubereitung zur intravenösen Applikation sowie Verfahren zu ihrer Herstellung**
Pharmaceutical composition for intravenous administration containing cyclosporin and method for its preparation
Composition pharmacéutique pour administration intraveneuse contenant de la cyclosporine et méthode pour sa préparation

(30) Priorität: 18.05.1992 DE 4216373
(43) Veröffentlichungstag der Anmeldung: 24.11.1993
(73) Patentinhaber: CicloMulsion AG, 76229 Karlsruhe (DE)
(72) Erfinder: Dietl, Hans, Dr., 83043 Bad Aibling (DE)
(74) Vertreter: Reinhard - Skuhra - Weise & Partner

(56) Entgegenhaltungen:
- EP-A- 0 361 928
- DATABASE WPI Section Ch, Week 8651, Derwent Publications Ltd., London, GB; Class B05, AN 86-335072
- DATABASE WPI Section Ch, Week 9243, Derwent Publications Ltd., London, GB; Class B05, AN 92-352740
- SCAND. J. IMMUNOL. Bd. 35, Nr. 2, Februar 1992, Seiten 231 - 236 A. TIBELL 'Cyclosporine A in fat emulsion carrier: Immunosuppressive effect in vitro.'
- BIOPHARM. DRUG DISPOS. Bd. 12, Nr. 6, 1991, Seiten 457 - 466 A. K. SHAH 'Effect of co-administration of Intralipid (TM) on the pharmacokinetics of cyclosporine in the rabbit.'
- J. PHARM. SCI. Bd. 79, Nr. 3, 1990, Seiten 216 - 219 S. VENKATARAM 'Pharmacokinetics of two alternative dosage forms of cyclosporine: Liposomes and Intralipid.'
- J. MICROENCAPSULATION Bd. 6, Nr. 2, 1989, Seiten 161 - 164 A. YANAGAWA 'Selective transfer of cyclosporin of thoracic lymphatic systems by the application of lipid microspheres.'
- TRANSPLANTATION Bd. 29, Nr. 5, 1980, Seiten 361 - 366 W.P. HOMAN 'Studies on the immunosuppressive properties of cyclosporin A in rats receiving renal allografts.'
- DRUGS EXP. CLIN. RES. Bd. 11, Nr. 9, 1985, Seiten 595 - 600 Y. MIZUSHIMA 'Lipid microspheres as novel drug carriers.'

## Beschreibung

Die Erfindung betrifft eine neue, Cyclosporin(e) enthaltende pharmazeutische Zubereitung, ein Herstellungsverfahren für diese pharmazeutische Zubereitung und ihre Verwendung zur intravenösen Applikation.

Cyclosporine sind von Wissenschaftlern der Sandoz AG, Basel entdeckte cyclische Oligopeptide aus niederen Pilzen. Insbesondere Cyclosporin A bzw. Cyclosporin G werden als Immunsuppressiva, insbesondere bei Organ-Transplantationen, eingesetzt. Weiterhin bevorzugt wird das Cyclosporin-Derivat "SDZ IMM 125", ein Hydroxyethylderivat von D-Serin-8-Cyclosporin. Auch die Anwendung bei anderen Erkrankungen, z.B. Diabetes und Psoriasis sowie zahlreichen Autoimmunerkrankungen (z.B. rheumatische Arthritis, endogene Uveitis etc.), wurde beschrieben.

Das bekannteste Cyclosporin ist Cyclosporin A ( Formel: C₆₂H₁₁₁N₁₁O₁₂).

Aus Pilzen wird Cyclosporin A durch Säulenchromatographie über Kieselgel als weißes amorphes Pulver gewonnen, es kristallisiert aus Aceton in weißen Nadeln mit einem Schmelzpunkt von 148 - 151°C. Neben dem Cyclosporin A sind zahlreiche andere Cyclosporine bekannt, die von Cyclosporin A bis Cyclosporin Z reichen (Römpps Chemie Lexikon, 9. Auflage, Seiten 841 - 843). Auch halbsynthetische Derivate des Cyclosporins sind bekannt und erfindungsgemäß einsetzbar. Es handelt sich dabei um chemisch einander sehr ähnliche Substanzen, die aus cyclischen Polypeptiden aus 11 zum Teil methylierten Aminosäuren bestehen. Cyclosporine sind löslich in Alkohol, Äther, Aceton und chlorierten Kohlenwasserstoffen und natürlichen Ölen (Triglyceriden von Fettsäuren).

J. Microencapsulation 6(2): 161-164 (1989) beschreibt auf Seite 162, 1. Abs., die Bildung einer Mischung aus Cyclosporin A mit Olivenöl und Eilecithin, welche mit Glycerin und Wasser aufgefüllt und unter Verwendung eines Homogenisators (Polytron) emulgiert wird. Anschließend wird die Mischung über ein 0,45 µm Filter filtriert.

In Transplantation 29(5): 361-366 (1980) ist auf Seite 361 unter der Überschrift "Cyclosporin A" die Herstellung und Verabreichung von Cyclosporin A beschrieben. Cyclosporin A wird in Olivenöl aufgelöst, was durch Rühren für 16 Stunden bei 37°C erreicht wird.

In einem weiteren Dokument (J.Pharm. Sci. 79(3):216-219 (1990), Seiten 216 und 217) werden Cyclosporin enthaltende Liposomen sowie Dispersionen fester Partikel von Cyclosporin beschrieben. Die Bildung einer Fettemulsion ist nicht beschrieben.

In Derwent File WPI AN 86-335072 (1986) und JP-A-61 249 918 sind Cyclosporin enthaltende Augentropfen offenbart.

Cyclosporin A ist zur oralen Anwendung als Lösung zum Einnehmen gelöst in einer Mischung von Alkohol mit einem Pflanzenöl beschrieben und im Verkehr (Pharmakopoeia Martindale, 29th Edition, US Pharm. XXII, 619).

Neben der oralen Einnahme ist insbesondere auch die intravenöse Verabreichung von Cyclosporin A von Bedeutung, da vor allem unmittelbar nach Organ-Transplantationen eine orale Verabreichung nicht möglich ist. Da Cyclosporine in wäßrigem Medium unlöslich sind, wird zur intravenösen Verabreichung das Cyclosporin in einem Gemisch aus Alkohol und Poly (oxyethylen)-40-Rizinusöl gelöst, vor der Anwendung mit Kochsalzlösung oder Glucose-Lösung verdünnt und langsam infundiert.

In EP-A-361 928 ist eine Zusammensetzung in Form einer Emulsion beschrieben, wobei die zur Emulgierung zwingend notwendigen Tenside unter anderem Polyoxyethylene, wie beispielsweise Polyoxyethylen-Rizinus-Öle, sind.

In Biopharm. Drug. Dispos. 12(6): 457-466 (1991) ist die Verwendung von Intralipid zur Herstellung einer Fettemulsion mit Cyclosporin beschrieben. Dabei wird Cyclosporin A in Ethanol gelöst.

Scand.J. Immunol. 35 (2): 231-236 (1992) beschreibt die Verwendung einer Fettemulsion als Träger zur intravenösen Verabreichung von Cyclosporin A. Die dafür verwendeten Phospholipide sind nicht näher spezifiziert.

In der United States Pharmacopoeia XXII, 619) wird ein Cyclosporin-Konzentrat zur Injektion beschrieben, bei dem es sich um eine sterile Lösung von Cyclosporin in einer Mischung von Alkohol mit einem geeigneten Pflanzenöl handeln soll. Diese Darreichungsform ist für eine Injektion nicht nur ungeeignet, sondern sogar letal, da es bei Injektion eines Öles in dieser Form sofort zu einer lebensgefährlichen Embolie kommen kann.

Daher befindet sich die intravenös anwendbare Form von Cyclosporin A nur in Form des obigen Konzentrats mit Alkohol und Poly(oxyethlen)-40-Rizinusöl im Verkehr als Sandimmun®-Infusions-Konzentrat. Die zur Lösung von Cyclosporin A verwendeten Hilfsstoffe Alkohol und Poly(oxyethylen)-40-Rizinusöl sind nicht nur keine idealen, sondern sogar gefährliche Hilfsstoffe. Durch den Gehalt an Alkohol besteht (siehe Fachinformation Sandimmun®) ein gesundheitliches Risiko unter anderem bei Leberkranken, Alkoholkranken, Epileptikern, Hirngeschädigten, Schwangeren und Kindern. Poly(oxyethylen)-40-Rizinusöl in Injektions- und Infusionslösungen kann insbesondere bei Personen mit allergischer Krankheitsbereitschaft oder bei Personen, die kürzlich schon ein Poly(oxyethylen)-40-Rizinusöl-haltiges Präparat als Injektion oder Infusion erhalten haben, zu Überempfindlichkeitsreaktionen führen, die sich als Blutdruckabfall, Mangeldurchblutung mit Blaufärbung der Lippen und Fingernägel, Luftnot sowie Hitzeanwallung mit zeitlichem Erröten der Haut äußern können. Diese Reaktionen können zeitlich wie auch in ihrem Ausmaß unterschiedlich verlaufen und auch zu lebensbedrohlichen Zuständen führen. Außerdem kann bei längerdauernder Anwendung aufgrund des Poly(oxyethylen)-40-Rizinusöl-Gehaltes eine Erhöhung der Blutfettwerte mit krankhafter Verschiebung des Lipoproteinmusters, eine Beeinträchtigung der Fließeigenschaften des Blutes und der Aggregationsfährigkeit der roten Blutkörperchen auftreten. (Fachinformation von Sandimmun®; Achtung bei Poly(oxyethylen)-40-Rizinusöl in Injektions- und Infusionslösungen, Deutsche Apothekerzeitung 125, 769 (1985), Pharmakopoeia Martindale, 29th Edition (1989), 614 - 619). Poly(oxyethylen)-40-Rizinusöl (z.B. Cremophor® EL) ist ein nichtionischer Emulgator, der durch Umsetzung von 35 Molen Ethylenoxid mit 1 Mol Rizinusöl hergestellt wird. Cremophor EL fällt nicht unter die natürlichen Öle und ist - im Gegensatz zu diesen - in Wasser löslich.

Daher ist es von großer Bedeutung, Cyclosporin-haltige Injektionslösungen bzw. Infusionslösungen zur Verfügung zu haben, die weder Alkohol noch Poly(oxyethylen)-40-Rizinusöl enthalten und die trotzdem Cyclosporin in therapeutisch ausreichender Menge enthalten sowie außerdem gut verträglich sind und ohne die oben beschriebenen Nebenwirkungen verabreicht werden können.

Obwohl diese Probleme seit mehr als zehn Jahren bekannt sind, ist es trotz aller Bemühungen bisher nicht gelungen, eine geeignete Injektionslösung bzw. Infusionslösung mit Cyclosporinen zur intravenösen Anwendung bereitzustellen.

Eine Aufgabe der vorliegenden Erfindung ist daher, eine Cyclosporin(e) enthaltende pharmazeutische Zubereitung bereitzustellen, welche weder Alkohol noch Poly(oxyethylen)-40-Rizinusöl enthält, und die zur intravenösen Anwendung geeignet ist.

Diese Aufgabe wird erfindungsgemäß dadurch gelöst, daß eine Cyclosporin(e) enthaltende pharmazeutische Zubereitung in Form einer Emulsion zur intravenösen Verabreichung bereitgestellt wird, dadurch gekennzeichnet, daß sie
a) ein oder mehrere Cyclosporin(e),
b) ein oder mehrere natürliche Öle,
c) 3-sn-Phosphatidylcholin und/oder Phosphatidylethanolamin,
d) pharmazeutisch verträgliche Alkalisalze freier Fettsäuren, und
e) Wasser enthält,
wobei nicht-ionische Tenside und Ethanol nicht enthalten sind.

Die vorliegende Erfindung löst somit überraschenderweise das Problem der oben beschriebenen Nebenwirkungen, indem eine Cyclosporin-haltige Injektionslösung bzw. Infusionslösung hergestellt wird, in welcher das Cyclosporin in therapeutisch ausreichender Konzentration in einer intravenös anwendbaren Mischung aus natürlichen Triglyceriden und Wasser und 3-sn-Phosphatidylcholinen und/oder Phosphatidylethanolamin und pharmazeutisch verträglichen Alkalisalzen freier Fettsäuren enthalten ist.

Ein weiterer völlig überraschender Vorteil - neben der Abwesenheit der unverträglichen Hilfsstoffe Alkohol und Poly(oxyethylen)-40-Rizinusöl - besteht in ihrer erhöhten therapeutischen

Wirksamkeit mit der erfindungsgemäßen pharmazeutischen Zubereitung. Damit kann die Dosis an Cyclosporin(en) vermindert werden und dementsprechend Häufigkeit und Schwere der Nebenwirkungen. Eine mögliche Erklärung hierfür könnte sein, daß das Cyclosporin in der erfindungsgemäßen Darreichungsform in einer Mikrosphäre eines Fettpartikels gelöst ist und dieses Fettpartikel mit dem darin inkorporierten Cyclosporin in einem wesentlichen Umfang direkt an den Ort der Wirksamkeit des Cyclosporins, die Lymphozyten, gelangt. Erst dort wird dann wahrscheinlich die Mikrosphäre des bis dahin intakten Fettpartikels aufgespalten und das Cyclosporin direkt und vollständig am Wirkungsort freigesetzt, während im Gegensatz dazu bei der bisherigen üblichen Darreichungsform Cyclosporin unspezifisch im ganzen Organismus verteilt wird. Bei zahlreichen möglichen Einsatzmöglichkeiten, z.B. bei Diabetes, rheumatischer Arthritis, Psoriasis, andere Autoimmunerkrankungen ist zwar Cyclosporin wirksam, aber wegen der dosisabhängigen Nebenwirkungen in der Anwendung begrenzt. Die erfindungsgemäße Cylosporin(e) enthaltende pharmazeutische Zubereitung schafft die Möglichkeit, Cyclosporin direkt und gezielt an seinen eigentlichen "Wirkungsort", das Immunsystem, zu bringen, wodurch sich die Anwendungsmöglichkeiten erheblich vergrößern können.

Die erfindungsgemäße pharmazeutische Zubereitung zur intravenösen Applikation enthält eine therapeutische Menge eines Cyclosporins oder mehrerer Cyclosporine, ein pharmazeutisch verträgliches Öl, 3-sn-Phosphatidylcholin und ein Alkalisalz einer freien Fettsäure sowie eine Substanz zur Isotonierung des Gemisches, z.B. Glycerin und/oder Sorbit und/oder Xylit.

Intravenös verabreichbare Fettemulsionen zur parenteralen Ernährung sowie Verfahren zu ihrer Herstellung sind aus dem Stand der Technik bekannt (z.B. Deutsche Patentschrift 1249454, British Patent 2406621, DOS 3721137, Europäische Patentschrift 071995, Deutsche Offenlegungsschrift 3032300). In diesen Druckschriften wird die Herstellung von Fettemulsionen unter Verwendung von natürlichen ölen und Phosphatidylcholinen als Emulgator beschrieben.

Diese Fettemulsionen sind Öl-in-Wasser-Emulsionen, bei denen die Tröpfchengröße der einzelnen Fett-Tröpfchen unter 4 Mikron liegt, damit diese Emulsionen ohne Gefahr einer Embolie infundiert werden können.

Als Öle werden Sojaöl und/oder Distelöl und/oder MCT-Öle (mittelkettige Triglyceride - Kokosöl) verwendet, die Phosphatidylcholine in Form von Sojalecithin und bevorzugt Eilecithin. Die Öle werden in einer wäßrigen Lösung emulgiert, wonach man das Gemisch zu einer Emulsion mit einer Partikelgröße von weniger als 4 Mikron homogenisiert (Deutsche Patentschrift 1249454).

Da Cyclosporine in natürlichen Ölen löslich sind, könnte man es als naheliegend betrachten, Cyclosporine einfach in solchen handelsüblichen Fettemulsionen (Handelsnamen: Intralipid®, Lipovenös®, Liposyn®) durch Zugabe von kristallisiertem Cyclosporin zu lösen. Dies gelingt jedoch nicht, da selbst nach intensivem Rühren der weitaus überwiegende Teil des Cyclosporins immer noch in Form ungelöster Kristalle vorliegt. Dies dürfte darauf zurückzuführen sein, daß das Cyclosporin die Lipid-Hülle nicht durchdringen kann, d.h. ein "Beladen" der Fetttröpfchen der Fettemulsionen nicht möglich ist. Daher erhält man sowohl eine völlig ungenügende, therapeutisch nicht ausreichende Konzentration des Cyclosporins in der Lösung sowie feste Teilchen in der Lösung, die sich bei intravenöser Applikation unter Umständen für den Patienten lebensbedrohlich auswirken können. Obwohl seit langer Zeit die Schädlichkeit der in dem Cyclosporin-haltigen Konzentrat enthaltenen Hilfsstoffe Alkohol und Poly(oxyethylen)-40-Rizinusöl bekannt ist, gelang es bisher nicht, das Problem zu lösen.

Es wurde nun überraschenderweise erfindungsgemäß gefunden, daß es sehr wohl möglich ist, eine intravenös anwendbare Applikationsform von Cyclosporin(en) ohne Alkohol und ohne Poly(oxyethylen)-40-Rizinusöl in Form einer Emulsion von Cyclosporinen in natürlichen, pharmazeutisch verträglichen ölen, 3-sn-Phosphatidylcholin und Wasser herzustellen. Entscheidend ist dabei, daß das Cyclosporin zunächst vollständig in dem verwendeten Öl gelöst wird, anschließend unter Verwendung von 3-sn-Phosphatidycholin, bevorzugt in Form von Eilecithin, emulgiert wird, wobei vor der Emulgierung Alkalisalze freier Fettsäuren zugegeben werden oder in dem Gemisch aus Cyclosporinen, natürlichen Ölen, 3-sn-Phosphatidylcholin und/oder Phosphatidylethanolamin freie natürliche Fettsäuren und/oder Alkalisalze freier Fettsäuren enthalten sind und vor der Emulgierung eine Alkalilauge zugegeben wird, und das Gemisch zu einer Emulsion mit einer Partikelgröße von weniger als 4 Mikron homogenisiert wird, bevorzugt im Mittel zwischen 0,2 - 0,7 µm.

Nach dem anschließenden Sterilisieren erhält man eine stabile Injektionslösung bzw. Infusionslösung mit einer therapeutisch anwendbaren Konzentration an Cyclosporin(en).

Als Cyclosporine können natürliche und synthetische Cyclosporine, beispielsweise die bekannten Cyclosporine A - Z verwendet werden; bevorzugt sind Cyclosporin A und Cyclosporin G sowie das Cyclosporin-Derivat SDZ IMM 125.

Als natürliche Öle können verwendet werden Sojaöl, Distelöl (Safloröl), Kokosöl (MCT-Öle), Fischöle, Maiskeimöl, Olivenöl etc.. Bevorzugt ist Sojaöl. Das verwendete Öl muß möglichst frei von Peroxiden sein. Das aus dem Stand der Technik bekannte Poly(oxyethylen)-40-Rizinusöl ist ein synthetisches Derivat und kann erfindungsgemäß nicht verwendet werden.

Das verwendete Cyclosporin, z.B. Cyclosporin A, wird zunächst in dem Öl, z.B. Sojaöl, gelöst, wobei im allgemeinen die Konzentration des Cyclosporins im Öl zwischen 0,2 - 7,0 Gewichtsprozent, bevorzugt zwischen 1 - 5 Gewichtsprozent, liegt. Das Lösen geschieht in bekannter Weise durch Einrühren des Cyclosporins in das Öl. Es sollte darauf geachtet werden, daß dabei das Vorhandensein von Sauerstoff ausgeschlossen wird, um eine Oxidation zu vermeiden. Zu der Cyclosporin-haltigen, öligen Lösung wird nun sauerstoff-freies Wasser gegeben sowie als Emulgator 3-sn-Phosphatidylcholin, bevorzugt in Form von Eilecithin und/oder Sojalecithin. Auf 100 Teile Öl werden dabei im allgemeinen 4 - 20 Teile des 3-sn-Phosphatidylcholins gegeben.

Besonders bevorzugt ist Eilecithin mit einem Gehalt von 3-sn-Phosphatidylcholin von 60 - 85%. Das 3-sn-Phosphatidylcholin kann auch teilweise hydriert sein.

Als Lieferanten des 3-sn-Phosphatidylcholins, des teilhydrierten 3-sn-Phosphatidylcholins oder hydrierten 3-sn-Phosphatidylcholins werden Eilecithin und/oder Sojalecithin bevorzugt, insbesondere Eilecithin. Geeignet sind vor allem Lecithine mit einem Gehalt von mehr als 60% 3-sn-Phosphatidylcholin und/oder teilhydriertem 3-sn-Phosphatidylcholin und/oder hydriertem 3-sn-Phosphatidylcholin.

Außerdem wird ein Alkalisalz einer freien Fettsäure mit 6- 26 Kohlenstoffatomen zugegeben, um den pH-Wert auf 5- 9 einzustellen und die Emulgierung und spätere Homogenisierung zu erleichtern. Bevorzugt sind die Natrium- bzw. Kalisalze der Palmitinsäure, Palmitoleinsäure, Stearinsäure, Ölsäure, Linolsäure und Linolensäure.

Das Gemisch, welches nun Cyclosporin, natürliches Öl, 3-sn-Phosphatidylcholin, Wasser und ein Alkalisalz einer freien Fettsäure enthält, wird nun mit Wasser soweit verdünnt, daß das Öl 5- 40 % des Gesamtgewichts beträgt.

Es wird nun durch starkes Rühren, bevorzugt mit einem Ultra-Turrax, eine Rohemulsion hergestellt.

Um sicherzustellen, daß die Lösung blutisoton ist, kann gegebenenfalls Glycerin und/oder Sorbit und/oder Xylit in entsprechender Menge zugegeben werden.

Diese Rohemulsion wird nun in einem Hochdruck-Homogenisator bei Drucken zwischen 100 - 500 bar so lange, unter Umständen mehrmals, homogenisiert, bis man eine Emulsion erhält, bei der die Partikelgröße aller Partikel unter 4 Mikron liegt, bevorzugt unter 1,5 Mikron (98 % aller Teilchen).

Danach wird die Emulsion durch Zugabe von Wasser auf die gewünschte Konzentration verdünnt. Anschließend wird die Emulsion in geeignete Behältnisse, z.B. in Flaschen oder Ampullen, abgefüllt und hitzesterilisiert.

Üblicherweise und vorzugsweise liegt das in der intravenös anwendbaren Arzneiform enthaltene Cyclosporin in vollständig gelöster Form vor. Dabei ist das Cyclosporin, gelöst in Öl, im Inneren eines Fettpartikels enthalten, wobei das Öl mit einer Hülle, bestehend im wesentlichen aus Phospholipid (3-sn-Phosphatidylcholin), umgeben ist. Es ist jedoch auch möglich, das Cyclosporin bei der Herstellung in so hoher Konzentration anzuwenden, daß nur ein Teil des Cyclosporins gelöst vorliegt, ein anderer Teil sich jedoch in fester Form im Inneren des Fettpartikels befindet. Wegen der das Fettpartikel umgebenden Hülle, welche das feste Cyclosporin nicht durchdringen kann, kann somit sogar festes Cyclosporin infundiert werden.

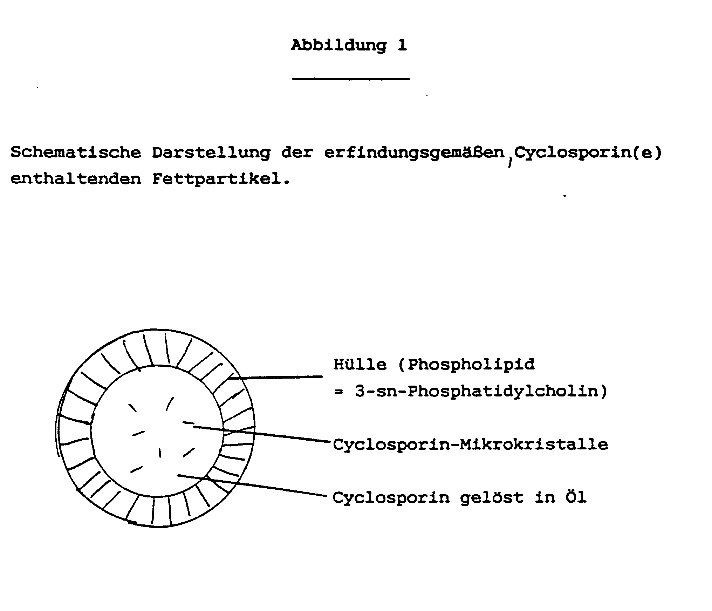

Die beiliegende Abbildung 1 zeigt in schematischer Form den Aufbau der erfindungsgemäßen, Cyclosporin enthaltenden Fettpartikel.

Die Herstellung solcher Arzneiformen geschieht nach dem erfindungsgemäßen Verfahren dergestalt, daß das erfindungsgemäß verwendete Öl auf beispielsweise ca. 50 - 70°C erwärmt und dann Cyclosporin in einer Menge zugegeben wird, so daß eine bei dieser höheren Temperatur fast gesättigte Lösung entsteht; dann wird entsprechend dem erfindungsgemäßen Verfahren die erfindungsgemäße intravenöse Arzneiform hergestellt.

Bei längerer Lagerung bei Zimmertemperatur oder tieferen Temperaturen kann ein Teil des ursprünglich eingesetzten Cyclosporins ausfallen. Dieses ausgefallene mikrokristalline Cyclosporin bleibt jedoch im Fettpartikel und dringt nicht als fester Kristall in die wäßrige Phase ein. Damit lassen sich hochkonzentrierte erfindungsgemäße Arzneiformen herstellen, die insbesondere als vor der Anwendung zu verdünnende Konzentrate geeignet sind.

Die folgenden Ausführungsbeispiele beschreiben die erfindungsgemäße Mischung und das Verfahren zu ihrer Herstellung im einzelnen. Die Erfindung ist jedoch nicht hierauf beschränkt.

### Beispiel 1

10,0 kg winterisiertes Sojaöl (neutraler pH-Wert, peroxidfrei) wird mit Stickstoff begast und unter Rühren und weiterem Begasen mit Stickstoff auf ca. 50°C erwärmt. Unter winterisiertem Sojaöl wird Sojaöl verstanden, das vor der Anwendung auf unter -10°C abgekühlt wird, damit in der Kälte unlösliche Anteile ausfallen, die abfiltriert werden.

Zu diesem Sojaöl werden nun 400 g Cyclosporin A gegeben und unter Rühren gelöst.

In ein zweites Gefäß werden 2,5 kg Glycerin gegeben sowie 10 1 Wasser. Es wird mit Stickstoff begast, bis der Sauerstoffgehalt unter 0,5 mg/l liegt.

Es werden 1,2 kg Eilecithin (Jodzahl 60 - 70) mit einem Gehalt von ca. 80 % 3-sn-Phosphatidylcholin und ca. 12 % Phosphatidylethanolamin (Kephalin) zugegeben sowie 40 g Natriumoleat.

Durch starkes Rühren mit einem Ultra-Turrax wird bei einer Temperatur von ca. 50°C eine Rohemulsion hergestellt.

Nun wird das Sojaöl mit dem darin gelösten Cyclosporin A durch ein fettresistentes Entkeimungsfilter mit einer Porengröße von ca. 50 Mikron in dieses zweite Gefäß überführt und weitere 10 Minuten bei ca. 50°C mit einem Ultra-Turrax stark gerührt. Der pH-Wert wird überprüft und sollte zwischen 5 und 9 liegen bevorzugt zwischen 7,0 - 8,5. Ist dies nicht der Fall, wird gegebenenfalls noch etwas Natriumoleat zugegeben.

Die erhaltene Rohemulsion wird durch ein Edelstahlfilter (Porengröße zwischen 5 - 50 Mikron) filtriert und anschließend mit einem 2-Stufen-Hochdruck-Homogenisator mit 3 Kolben homogenisiert (1. Stufe 100 bar, 2. Stufe 400 bar). Der erforderliche Homogenisierdruck wird dabei mit Wasser für Injektionszwecke aufgebaut, nicht mit der Rohemulsion, um Verschmutzung und Abscheidung von Öltröpfchen zu vermeiden.

Der Vorgang der Homogenisierung wird so oft wiederholt, bis die gewünschte Partikelgröße erreicht ist. Kein Partikel darf größer als 4 Mikron sein. Im Mittel beträgt der Durchmesser der Partikel 0,2 - 0,6 Mikron.

Um eine gut anwendbare Partikelgröße zu erreichen, ist es im allgemeinen nötig, den Vorgang der Homogenisierung vier Mal zu wiederholen. Nach jedem Homogenisierungsvorgang sollte die Emulsion auf 30 - 50°C abgekühlt werden.

Die Emulsion wird in 57 l sauerstoff-freies Wasser ad injektabilia gegeben. Es wird nochmals mit Stickstoff begast, bis der Sauerstoffgehalt unter 0,5 mg/l liegt.

18,5 l Wasser ad injectabilia werden als Nachwasser bei 400 bar durch den Homogenisator gegeben und dann zu obiger Gesamtmenge zugefügt. Der gesamte Ansatz wird weiter mit Stickstoff begast, bis der Sauerstoffgehalt unter 0,5 mg/l liegt. Vor der Abfüllung in Glasflaschen wird durch ein Edelstahlfilter mit einer mittleren Porengröße von 5 Mikron filtriert. Dabei sollte der Filtrationsdruck 0,2 bar nicht übersteigen, damit ein Brechen der Emulsion verhindert wird.

Die Partikelgröße und die Partikelverteilung werden unter einem Mikroskop oder einem Coulter Counter bestimmt. Die Partikelverteilung beträgt:

| Partikelgröße | Zahl der Teilchen |
|---|---|
| < 0,2 Mikron | 34 % |
| 0,2 - 0,5 Mikron | 43 % |
| 0,5 - 0,9 Mikron | 14 % |
| 0,9 - 1,2 Mikron | 6 % |
| 1,2 - 1,9 Mikron | 2 % |
| 1,9 - 2,2 Mikron | unter 1 % |
| 2,2 - 2,5 Mikron | unter 1 % |
| 2,5 - 3,2 Mikron | unter 1 % |
| > 3,2 Mikron | unter 1 % |

Die Emulsion wird in 100 ml-Glasflaschen abgefüllt. Die Flaschen werden vor der Abfüllung mit Stickstoff begast. Dabei ist es vorteilhaft, den verwendeten Stickstoff auf minus 20 - 30°C abzukühlen, damit der Stickstoff leichter auf den Boden der Flasche sinkt. Auch während des Abfüllvorganges sollten die Flaschen weiterhin mit Stickstoff begast werden.

Die erhaltene Emulsion wird hitzesterilisiert bei 121°C für 20 Minuten. Vorteilhaft wird dabei ein sogenannter Rotationsautoklav verwendet, in dem die Flaschen während der Sterilisation langsam über Kopf rotieren. Dadurch wird die zum Abfüllen und Abkühlen notwendige Zeit vermindert und vermieden, daß sich die Partikelgröße ändert. Man erhält eine sterile, intravenös anwendbare Emulsion mit einem Gehalt von 400 mg Cyclosporin A/100 ml. Die Partikelverteilung nach der Sterilisation ist wie folgt angegeben:

| Partikelgröße | Zahl der Teilchen |
|---|---|
| < 0,2 Mikron | 25 % |
| 0,2 - 0,5 Mikron | 46 % |
| 0,5 - 0,9 Mikron | 19 % |
| 0,9 - 1,2 Mikron | 6 % |
| 1,2 - 1,5 Mikron | 2 % |
| 1,5 - 1,9 Mikron | unter 1 % |
| 1,9 - 2,2 Mikron | unter 1 % |
| 2,2 - 3,2 Mikron | unter 1 % |
| > 3,2 Mikron | 0 % |

Die so erhaltene sterile Emulsion kann langsam über 2- 6 Stunden bei Patienten infundiert werden, wobei dann insgesamt 400 mg Cyclosporin A als therapeutisch optimale Höchstdosis infundiert wird.

Die Infusionslösung enthält pro 100 ml:

| | |
|---|---|
| Cyclosporin A: | 400 mg |
| Sojaöl: | 10 g |
| Eilecithin: | 1,2 g |
| Glycerin: | 2,5 g |
| Natriumoleat: | 40 mg |
| sowie Wasser ad | 100 ml |

### Beispiel 2

Das Verfahren des Beispiels 1 wird wiederholt, wobei statt des Sojaöls ein Fischöl-Konzentrat, das 18 % Eicosapentaensäure und 12 % Docosahexaensäure enthält, verwendet wird. Man erhält eine pharmazeutische Zubereitung zur intravenösen Applikation mit der folgenden Zusammensetzung pro 100 ml:

| | |
|---|---|
| Cyclosporin A: | 400 mg |
| Fischöl-Konzentrat : | 10 g |
| Eilecithin: | 1,2 g |
| Glycerin: | 2,5 g |
| Natriumoleat: | 40 mg |
| Wasser ad injectabilia ad | 100 ml |

| Partikelgröße | Zahl der Teilchen |
|---|---|
| 0,2 Mikron | ca. 25 % |
| 0,2 - 0,5 Mikron | ca. 46 % |
| 0,5 - 0,9 Mikron | ca. 19 % |
| 0,9 - 1,2 Mikron | ca. 6 % |
| 1,2 - 1,5 Mikron | ca. 2 % |
| 1,5 - 1,9 Mikron | unter 1 % |
| 1,9 - 2,2 Mikron | unter 1 % |

### Beispiel 3

Das Verfahren des Beispiels 1 wird wiederholt, wobei jedoch pro 10 kg Sojaöl 250 g Cyclosporin A eingewogen werden.

Man erhält eine pharmazeutische Zubereitung zur intravenösen Applikation mit der folgenden Zusammensetzung pro 100 ml:

| | |
|---|---|
| Cyclosporin A: | 250 mg |
| Sojaöl: | 10 g |
| Eilecithin: | 1,2 g |
| Glycerin: | 2,5 g |
| Natriumoleat: | 40 mg |
| Wasser ad injectabilia ad | 100 ml |

| Partikelgröße | Zahl der Teilchen |
|---|---|
| 0,2 Mikron | ca. 24 % |
| 0,2 - 0,5 Mikron | ca. 58 % |
| 0,5 - 0,9 Mikron | ca. 16 % |
| 0,9 - 1,2 Mikron | ca. 2 % |
| 1,2 - 1,5 Mikron | unter 1 % |
| 1,5 - 1,9 Mikron | unter 1 % |

### Beispiel 4

Das Beispiel 1 wird wiederholt, wobei statt des in Beispiel 1 verwendeten Eilecithins ein Eilecithin mit einem Gehalt an 3-sn-Phosphatidylcholin von ca. 70% und einem Gehalt an Kephalin von ca. 20 % verwendet wird. Statt 40 mg Natriumoleat werden 50 mg Natriumoleat verwendet. Man erhält eine pharmazeutische Zubereitung zur intravenösen Anwendung mit der folgenden Zusammensetzung pro 100 ml:

| | |
|---|---|
| Cyclosporin A: | 400 mg |
| Sojaöl: | 10 g |
| Eilecithin: | 1,2 g |
| Glycerin: | 2,5 g |
| Natriumoleat: | 50 mg |
| Wasser ad injectabilia ad | 100 ml. |

| Partikelgröße | Zahl der Teilchen |
|---|---|
| 0,2 Mikron | ca. 30 % |
| 0,2 - 0,5 Mikron | ca. 49 % |
| 0,5 - 0,9 Mikron | 17 % |
| 0,9 - 1,2 Mikron | 3 % |
| 1,2 - 1,5 Mikron | unter 1 % |
| 1,5 - 1,9 Mikron | unter 1 % |

### Beispiel 5

Das Beispiel 1 wird wiederholt, wobei anstelle des Eilecithins ein teilhydriertes Eilecithin mit ca. 80 % teilhydriertem 3-sn-Phosphatidylcholin mit einer Jodzahl von 35 (Beispiel 1 : Jodzahl 60 - 70) eingesetzt wird sowie 50 g Natriumoleat. Man erhält eine pharmazeutiche Zubereitung zur intavenösen Anwendung mit folgender Zusammensetzung pro 100 ml.

| | |
|---|---|
| Cyclosporin A: | 400 mg |
| Sojaöl: | 10 g |
| Eilecithin (teilhydriert): | 1,2 g |
| Glycerin: | 2,5 g |
| Natriumoleat: | 50 mg |
| Aqua ad injectabilia ad | 100 ml |

| Partikelgröße | Zahl der Teilchen |
|---|---|
| 0,2 Mikron | ca. 10 % |
| 0,2 - 0,5 Mikron | ca. 63 % |
| 0,5 - 0,8 Mikron | ca. 23 % |
| 0,8 - 1,2 Mikron | ca. 4 % |
| 1,2 - 1,5 Mikron | unter 1 % |
| 1,5 - 1,9 Mikron | unter 1 % |
| 1,9 - 2,2 Mikron | unter 1 % |
| 2,2 - 3,2 Mikron | unter 1 % |
| > 3,2 Mikron | 0 % |

### Beispiel 6

Das Beispiel 1 wird wiederholt, wobei statt des Eilecithins Sojalecithin mit einem Gehalt von ca. 75 % 3-sn-Phosphatidylcholin eingesetzt wird.

### Beispiel 7

10,0 kg winterisiertes Sojaöl wird mit Stickstoff begast und unter Rühren auf ca. 50 - 60°C erwärmt. Eine Bestimmung der freien Fettsäuren im Sojaöl ergab einen Gehalt an freien Fettsäuren von 2,5 meq/l. Der pH-Wert liegt bei ca. 3,5 - 4,5.

Es werden 500 g Cyclosporin A zugegeben und unter Rühren gelöst. Es werden 10 l Wasser ad injectabilia, 2,5 kg Glycerin und 1,0 kg Eilecithin mit einem Gehalt an ca. 75 % 3-sn-Phosphatidylcholin bei einer Temperatur von ca. 50°C zugegeben. Es wird durch heftiges Rühren, bevorzugt mit einem Ultra-Turrax, eine Rohemulsion hergestellt. Es wird durch Zugabe einer 10 %igen Natriumhydroxidlösung ein pH-Wert von 7,5 - 8,5 eingestellt.

Die Rohemulsion wird durch ein Edelstahlfilter mit einer Porengröße von ca. 30 Mikron filtriert.

Anschließend wird mit einem 2-Stufen-Hochdruck-Homogenisator mit drei Kolben (1. Stufe 100 bar; 2. Stufe 450 bar) homogenisiert. Der erforderliche Homogenisierdruck wird mit Wasser ad injectabilia aufgebaut.

Der Homogenisierungsvorgang wird insgesamt vier Mal bei einer Temperatur von 30- 60°C durchgeführt.

Zu der Emulsion werden 10 l Wasser ad injectabilia gegeben und unter Rühren mit Stickstoff so lange begast, bis der Sauerstoffgehalt kleiner als 0,5 mg/l beträgt. 6,5 1 Wasser ad injectabilia werden nun als Nachwasser bei 450 bar durch den Homogenisator gegeben und zur Gesamtmenge zugefügt. Es wird weiter mit Stickstoff begast, bis der Sauerstoffgehalt unter 0,5 mg/l liegt.

Es wird durch ein Edelstahlfilter mit einer mittleren Porenweite von 5 Mikron filtriert, wobei darauf geachtet wird, daß der Filtrationsdruck nicht über 0,2 bar liegt.

Die Emulsion wird in Ampullen zu 20 ml abgefüllt und bei 120°C für 20 Minuten sterilisiert.

Eine Untersuchung der Partikelgröße ergab folgende Verteilung

| Partikelgröße | Zahl der Partikel |
|---|---|
| < 0,2 Mikron | 24 % |
| 0,2 - 0,5 Mikron | 58 % |
| 0,5 - 0,9 Mikron | 16 % |
| 1,2 - 1,5 Mikron | unter 1 % |
| 1,5 - 1,9 Mikron | unter 1 % |
| > 2,0 Mikron | 0 % |

Die erhaltene pharmazeutische Zubereitung zur intravenösen Anwendung enthält pro Ampulle (20 ml) 250 mg Cyclosporin A. Vor der Anwendung wird der Inhalt der Ampulle durch Zugabe zu einer handelsüblichen 10 %igen Fettemulsion im Verhältnis 1:5 bis 1:10 verdünnt und langsam infundiert.

### Beispiel 8

Das Beispiel 7 wird wiederholt, wobei jedoch statt 500 g Cyclosporin A, 250 g Cyclosporin A plus 250 g Cyclosporin G eingesetzt werden.

Eine Ampulle zu 20 ml enthält dann 125 mg Cyclosporin A und 125 mg Cyclosporin G.

### Beispiel 9

Eine Cyclosporin enthaltende pharmazeutische Zubereitung wird, wie im Beispiel 1 beschrieben, hergestellt, wobei jedoch statt des Cyclosporins A 150 g von SDZ IMM 125, ein Hydroxyethylderivat des D-Serin-8-Cyclosporins, eingesetzt wird. Die Herstellung und die Struktur von SDZ IMM 125 sind beschrieben in G. Baumann et al., Transplantation Proceedings Volume 24, No. 4, Suppl. 2, (1992), Seite 43-48, sowie P.C. Hiestand et al., Transplantation Proceedings Vol. 24, No. 4, Suppl. 2, Seite 31-38 (1992). Man erhält eine sterile, intravenös anwendbare Emulsion mit einem Gehalt von 150 mg des beschriebenen Cyclosporin-Derivats pro 100 ml Emulsion.

## Patentansprüche

1. Cyclosporin(e) enthaltende pharmazeutische Zubereitung in Form einer Emulsion zur intravenösen Verabreichung,
dadurch gekennzeichnet, daß sie
a) ein oder mehrere Cyclosporin(e),
b) ein oder mehrere natürliche Öle,
c) 3-sn-Phosphatidylcholin und/oder Phosphatidylethanolamin,
d) pharmazeutisch verträgliche Alkalisalze freier Fettsäuren, und
e) Wasser enthält,
wobei nicht-ionische Tenside und Ethanol nicht enthalten sind.

2. Cyclosporin(e) enthaltende pharmazeutische Zubereitung nach Anspruch 1,
dadurch gekennzeichnet,
daß sie in sterilisierte Form vorliegt.

3. Cyclosporin(e) enthaltende pharmazeutische Zubereitung nach Anspruch 1 oder 2,
dadurch gekennzeichnet,
daß das Cyclosporin ein natürliches und/oder ein synthetisches Cyclosporin und/oder ein Cyclosporin-Derivat ist, bevorzugt ein Cyclosporin A und/oder ein Cyclosporin G und/oder das Cyclosporin-Derivat SDZ IMM 125.

4. Cyclosporin(e) enthaltende pharmazeutische Zubereitung nach einem oder mehreren der vorhergehenden Ansprüche,
dadurch gekennzeichnet,
daß das 3-sn-Phosphatidylcholin in Form von 3-sn-Phosphatidylcholin-enthaltenden Substanzen vorliegt und daß vorzugsweise die 3-sn-Phosphatidylcholin-enthaltende Substanz Eilecithin oder Sojalecithin (Sojaphosphatid) ist.

5. Cyclosporin(e) enthaltende pharmazeutische Zubereitung nach einem oder mehreren der vorhergehenden Ansprüche,
dadurch gekennzeichnet,
daß das verwendete 3-sn-Phosphatidylcholin teilweise hydriert oder vollständig hydriert ist und daß vorzugsweise das teilweise hydrierte 3-sn-Phosphatidylcholin in der Form von teilweise hydriertem Sojalecithin oder teilweise hydriertem Eilecithin verwendet wird, wobei vorzugsweise das Eilecithin mindestens 60 Gew.-% 3-sn-Phosphatidylcholin, bevorzugt zwischen 60 - 85 Gew.-%, enthält.

6. Cyclosporin(e) enthaltende pharmazeutische Zubereitung nach einem oder mehreren der vorhergehenden Ansprüche,
dadurch gekennzeichnet,
daß der Gehalt an 3-sn-Phosphatidylcholinen in der pharmazeutischen Zubereitung 0,3 - 4, bevorzugt 0,6 - 2,4 Gew.% beträgt und daß der Gehalt der die 3-sn-Phosphatidylcholine enthaltenden Substanzen in der pharmazeutischen Zubereitung 0,5 - 6,5, bevorzugt 1 - 4 Gew.%, beträgt.

7. Cyclosporin(e) enthaltende pharmazeutische Zubereitung nach einem oder mehreren der vorhergehenden Ansprüche,
dadurch gekennzeichnet,
daß als natürliche Öle Sojaöl und/oder Distelöl und/oder mittelkettige Trigylceride (z.B. Kokosöl), und/oder Maiskeimöl und/oder Weizenkeimöl und/oder Sonnenblumenöl und/-oder Olivenöl und/oder Fischöl verwendet werden und daß vorzugsweise das natürliche Öl weitgehend frei von Peroxiden ist.

8. Cyclosporin(e) enthaltende pharmazeutische Zubereitung nach einem oder mehreren der vorhergehenden Ansprüche,
dadurch gekennzeichnet,
daß die Konzentration der Cyclosporine im Öl zwischen 0,2 - 7,0 Gew.-%, bevorzugt zwischen 1 - 5 Gew.-%, liegt.

9. Cyclosporin(e) enthaltende pharmazeutische Zubereitung nach einem oder mehreren der vorhergehenden Ansprüche,
dadurch gekennzeichnet,
daß das natürliche Öl in einem Anteil von 5 - 40 %, bevorzugt in einem Anteil von 10 - 30 %, des Gesamtgewichts in der pharmazeutischen Zubereitung vorliegt.

10. Cyclosporin(e) enthaltende pharmazeutische Zubereitung nach einem oder mehreren der vorhergehenden Ansprüche,
dadurch gekennzeichnet,
daß pharmazeutisch verträgliche gesättigte und/oder ungesättigte Alkalisalze freier Fettsäuren, bevorzugt mit 6- 26 Kohlenstoffatomen, enthalten sind, wobei vorzugsweise als pharmazeutisch verträgliche Alkalisalze freier Fettsäuren, die Alkalisalze der Fettsäuren Palmitinsäure und/oder Palmitoleinsäure und/oder Linolsäure und/oder Linolensäure verwendet werden.

11. Cyclosporin(e) enthaltende pharmazeutische Zubereitung nach einem oder mehreren der vorhergehenden Ansprüche,
dadurch gekennzeichnet,
daß der Gehalt an Alkalisalzen freier Fettsäuren 0,01 - 0,15 Gew.-%, bevorzugt 0,02 - 0,06 Gew.-%, beträgt.

12. Cyclosporin(e) enthaltende pharmazeutische Zubereitung nach einem oder mehreren der vorhergehenden Ansprüche,
dadurch gekennzeichnet,
daß der Gehalt an Wasser in der pharmazeutischen Zubereitung 60 - 95 Gew.-% , bevorzugt 70 - 90 Gew.-%, beträgt.

13. Cyclosporin(e) enthaltende pharmazeutische Zubereitung nach einem oder mehreren der vorhergehenden Ansprüche,
dadurch gekennzeichnet,
daß die pharmazeutische Zubereitung zusätzlich Glycerin in einer solchen Menge enthält, die geeignet ist, um die pharmazeutische Zubereitung isoton zu machen.

14. Cyclosporin(e) enthaltende pharmazeutische Zubereitung nach einem oder mehreren der vorhergehenden Ansprüche,
dadurch gekennzeichnet,
daß die in den natürlichen Ölen gelösten Cyclosporine im Inneren von aus natürlichen Ölen bestehenden Fettpartikeln eingeschlossen, von einer Hülle aus 3-sn-Phosphatidylcholin und/oder Phosphatidylethanolamin umgeben und in Wasser emulgiert sind.

15. Verfahren zur Herstellung der Cyclosporin(e) enthaltenden pharmazeutischen Zubereitung nach einem oder mehreren der vorhergehenden Ansprüche,
**dadurch gekennzeichnet,**
daß die Cyclosporine in natürlichen Ölen gelöst und anschließend mit 3-sn-Phosphatidylcholin und/oder Phosphatidylethanolamin und Wasser in an sich bekannter Weise emulgiert werden , wobei vor der Emulgierung Alkalisalze freier Fettsäuren zugegeben werden oder in dem Gemisch aus Cyclosporinen, natürlichen Ölen, 3-sn-Phosphatidylcholin und/oder Phosphatidylethanolamin freie natürliche Fettsäuren und/oder Alkalisalze freier Fettsäuren enthalten sind und vor der Emulgierung eine Alkalilauge zugegeben wird.

16. Verfahren nach Anspruch 15,
dadurch gekennzeichnet,
daß die Cyclosporine vor der Emulgierung in dem natürlichen Öl vollständig gelöst und anschließend mit Hilfe von 3-sn-Phosphatidylcholinen und Alkalisalzen freier Fettsäuren in Wasser zu einer Rohemulsion emulgiert werden und das Gemisch anschließend zu einer Emulsion, bevorzugt mit einer Partikelgröße von weniger als 4 µm, insbesondere unter 1,5 Mikron, homogenisiert wird.

17. Verfahren nach einem oder mehreren der vorhergehenden Ansprüche,
dadurch gekennzeichnet,
daß das Gemisch aus Cyclosporinen, natürlichen ölen, 3-sn-Phosphatidylcholin und Wasser bereits 0,01 - 0,15 % an freien natürlichen Fettsäuren und/oder deren Alkalisalzen enthält und dieses Gemisch mittels einer Alkalilauge vor der Emulgierung auf einen pH-Wert von 5-10, bevorzugt 7 - 9, eingestellt wird.

18. Verfahren nach einem oder mehreren der vorhergehenden Ansprüche,
dadurch gekennzeichnet,
daß die Alkalisalze freier Fettsäuren verwendet werden, um die Emulsion auf einen pH-Wert von 5- 10, bevorzugt 7,0 - 9,0, einzustellen.

19. Verfahren nach einem oder mehreren der vorhergehenden Ansprüche,
dadurch gekennzeichnet,
daß zur Herstellung der pharmazeutischen Zubereitung im wesentlichen sauerstofffreie Lösungen verwendet werden.

20. Verfahren nach einem oder mehreren der vorhergehenden Ansprüche,
dadurch gekennzeichnet,
daß pharmazeutisch verträgliche gesättigte und /oder ungesättigte Alkalisalze der Fettsäuren verwendet werden.

21. Verfahren nach einem oder mehreren der vorhergehenden Ansprüche,
dadurch gekennzeichnet,
daß zusätzlich Glycerin in einer Menge verwendet wird, die geeignet ist, die pharmazeutische Zubereitung isoton zu machen.

22. Verfahren nach einem oder mehreren der vorhergehenden Ansprüche,
dadurch gekennzeichnet,
daß durch intensives Rühren zunächst eine Rohemulsion hergestellt wird und anschließend die endgültige Emulgierung in einem Hochdruck-Homogenisator bei Drücken zwischen 100 - 500 bar erfolgt, wobei vorzugsweise der Hochdruck-Homogenisator mindestens drei Kolben besitzt.

23. Verfahren nach einem oder mehreren der vorhergehenden Ansprüche,
dadurch gekennzeichnet,
daß die Sterilisation der pharmazeutischen Zubereitung durch Hitzesterilisation erfolgt.

24. Verwendung der pharmazeutischen Zubereitung nach einem oder mehreren der vorhergehenden Ansprüche zur intravenösen Injektion und/oder Infusion.

## Claims

1. A pharmaceutical preparation containing cyclosporine(s) in the form of an emulsion for intravenous application, characterized in that it contains
a) one or more cyclosporine(s),
b) one or more natural oils,
c) 3-sn-phosphatidyl choline and/or phosphatidyl ethanol amine,
d) pharmaceutically tolerable alkali salts of free fatty acids, and
e) water
wherein non-ionic tensides and ethanol are not contained.

2. A pharmaceutical preparation containing cyclosporine(s) according to claim 1, characterized in that it is present in sterilized form.

3. A pharmaceutical preparation containing cyclosporine(s) according to claim 1 or 2, characterized in that the cyclosporine is a natural and/or a synthetic cyclosporine and/or a cyclosporine derivative, preferably a cyclosporine A and/or a cyclosporine G and/or the cyclosporine derivative SDZ IMM 125.

4. A pharmaceutical preparation containing cyclosporine(s) according to one or several of the preceding claims, characterized in that the 3-sn-phosphatidyl choline is present in the form of substances containing 3-sn-phosphatidyl choline, and that preferably, the substance containing 3-sn-phosphatidyl choline is egg lecithin or soy bean lecithin (soy phosphatide).

5. A pharmaceutical preparation containing cyclosporine(s) according to one or several of the preceding claims, characterized in that the used 3-sn-phosphatidyl choline is partially hydrogenated or completely hydrogenated and that preferably the partially hydrogenated 3-sn-phosphatidyl choline is used in the form of partially hydrogenated soy lecithin or partially hydrogenated egg lecithin, wherein the egg lecithin preferably contains at least 60% by weight, preferably between 60 and 85% by weight, of 3-sn-phosphatidyl choline.

6. A pharmaceutical preparation containing cyclosporine(s) according to one or several of the preceding claims, characterized in that the content of 3-sn-phosphatidyl cholines in the pharmaceutical preparation is 0.3 to 4, preferably 0.6 to 2.4% by weight, and that the content of the substances containing the 3-sn-phosphatidyl cholines in the
pharmaceutical preparation is 0.5 to 6.5, preferably 1 to 4% by weight.

7. A pharmaceutical preparation containing cyclosporine(s) according to one or several of the preceding claims, characterized in that soy oil and/or safflower oil and/or medium-chain triglycerides (e.g. coconut oil) and/or corn oil and/or wheat germ oil and/or sunflower oil and/or olive oil and/or fish oil are used as natural oils and that the
natural oil is preferably essentially free from peroxides.

8. A pharmaceutical preparation containing cyclosporine(s) according to one or several of the preceding claims, characterized in that the concentration of cyclosporines in the
oil is between 0.2 and 7.0% by weight, preferably between 1 and 5% by weight.

9. A pharmaceutical preparation containing cyclosporine(s) according to one or several of the preceding claims, characterized in that the natural oil is present in a share of 5 to 40%, preferably in a share of 10 to 30%, of the total weight in the pharmaceutical
preparation.

10. A pharmaceutical preparation containing cyclosporine(s) according to one or several of the preceding claims, characterized in that pharmaceutically tolerable saturated and/or unsaturated alkali salts of free fatty acids are contained, preferably having 6 to 26 carbon atoms, wherein preferably the alkali salts of the fatty acids palmitic acid and/or palmitoleic acid and/or linolenic acid and/or linoleic acid are used as alkali salts of free fatty acids.

11. A pharmaceutical preparation containing cyclosporine(s) according to one or several of the preceding claims, characterized in that the content of alkali salts of free fatty acids is 0.01 to 0.15% by weight, preferably 0.02 to 0.06% by weight.

12. A pharmaceutical preparation containing cyclosporine(s) according to one or several of the preceding claims, characterized in that the content of water in the pharmaceutical preparation is 60 to 95% by weight, preferably 70 to 90% by weight.

13. A pharmaceutical preparation containing cyclosporine(s) according to one or several of the preceding claims, characterized in that, in addition, the pharmaceutical preparation contains glycerin in such an amount which is suited to make the pharmaceutical preparation isotonic.

14. A pharmaceutical preparation containing cyclosporine(s) according to one or several of the preceding claims, characterized in that the cyclosporines dissolved in the natural oils are enclosed in the interior of fatty particles consisting of natural oils and are surrounded by an envelope of 3-sn-phosphatidyl choline and/or phosphatidyl ethanol amine and are emulsified in water.

15. A process for the production of the pharmaceutical preparation containing cyclosporine(s) according to one or several of the preceding claims, characterized in that the cyclosporines are dissolved in natural oils and subsequently emulsified with 3-sn-phosphatidyl choline and/or phosphatidyl ethanol amine and water in a manner known per se, wherein alkali salts of free fatty acids are added prior to the emulsification, or free natural fatty acids and/or alkali salts of free fatty acids are contained in the mixture of cyclosporines, natural oils, 3-sn-phosphatidyl choline and/or phosphatidyl ethanol amine and an alkali lye is added prior to the emulsification.

16. A process according to claim 15, characterized in that the cyclosporines are completely dissolved in the natural oil prior to the emulsification and are subsequently emulsified in water to a crude emulsion by means of 3-sn-phosphatidyl cholines and alkali salts of free fatty acids, and the mixture is subsequently homogenized to an emulsion, preferably with a particle size of less than 4 µm, in particular of less than 1.5 microns.

17. A process according to one or several of the preceding claims, characterized in that the mixture of cyclosporines, natural oils, 3-sn-phosphatidyl choline and water already contains 0.01 to 0.15% of free natural fatty acids and/or their alkali salts, and this mixture is adjusted to a pH value of 5 to 10, preferably 7 to 9, by means of an alkali lye prior to emulsification.

18. A process according to one or several of the preceding claims, characterized in that the alkali salts of free fatty acids are used to adjust the emulsion to a pH value of 5 to 10, preferably of 7.0 to 9.0.

19. A process according to one or several of the preceding claims, characterized in that substantially oxygen-free solutions are used for producing the pharmaceutical preparation.

20. A process according to one or several of the preceding claims, characterized in that pharmaceutically tolerable, saturated and/or unsaturated alkali salts of the fatty acids are used.

21. A process according to one or several of the preceding claims, characterized in that glycerin is additionally used in an amount suited to make the pharmaceutical preparation isotonic.

22. A process according to one or several of the preceding claims, characterized in that first a crude emulsion is produced by means of thorough stirring, and the final emulsifying is subsequently carried out in a high-pressure homogenizer at pressures between 100 and 500 bar, wherein the high-pressure homogenizer preferably comprises at least three pistons.

23. A process according to one or several of the preceding claims, characterized in that the sterilization of the pharmaceutical preparation is carried out by means of heat sterilization.

24. Use of the pharmaceutical preparation according to one or several of the preceding claims for intravenous injection and/or infusion.

## Revendications

1. Composition pharmaceutique sous la forme d'une émulsion pour administration intraveineuse contenant une(des) cyclosporines, caractérisée en ce qu'elle contient :
a) une ou plusieurs cyclosporine (s) ;
b) une ou plusieurs huiles naturelles ;
c) de la 3-sn-phosphatidylcholine et/ou de la phosphatidyléthanolamine ;
d) des sels alcalins d'acide gras libres pharmaceutiquement acceptables ; et
e) de l'eau,
la composition ne comprenant ni tensioactifs non ioniques, ni éthanol.

2. Composition pharmaceutique contenant une(des) cyclosporine(s), selon la revendication 1, caractérisée en ce qu'elle se présente sous forme stérilisée.

3. Composition pharmaceutique contenant une(des) cyclosporine(s), selon la revendication 1 ou 2, caractérisée en ce que la cyclosporine est une cyclosporine naturelle et/ou synthétique et/ou un dérivé de cyclosporine, de préférence une cyclosporine A et/ou une cyclosporine G et/ou le dérivé de cyclosporine SDZ IMM 125.

4. Composition pharmaceutique contenant une(des) cyclosporine(s), selon une ou plusieurs des revendications précédentes, caractérisée en ce que la 3-sn-phosphatidylcholine se trouve sous forme de substances contenant de la 3-sn-phosphatidylcholine, et en ce que la substance contenant de la 3-sn-phosphatidylcholine est de préférence la lécithine d'oeuf ou la lécithine de soja (phosphatide de soja).

5. Composition pharmaceutique contenant une(des) cyclosporine(s), selon une ou plusieurs des revendications précédentes, caractérisée en ce que la 3-sn-phosphatidylcholine utilisée est partiellement ou totalement hydrogénée, et en ce que la 3-sn-phosphatidylcholine partiellement hydrogénée est utilisée de préférence sous la forme de lécithine de soja partiellement hydrogénée ou de lécithine d'oeuf partiellement hydrogénée, de préférence la lécithine d'oeuf contenant au moins 60 % en poids, de préférence entre 60 et 85 % en poids, de 3-sn-phosphatidylcholine.

6. Composition pharmaceutique contenant une(des) cyclosporine(s), selon une ou plusieurs des revendications précédentes, caractérisée en ce que la teneur en 3-sn-phosphatidylcholine de la composition pharmaceutique va de 0,3 à 4, de préférence de 0,6 à 2,4 % en poids, et en ce que la teneur de la composition pharmaceutique en les substances contenant de la 3-sn-phosphatidylcholine va de 0,5 à 6,5, de préférence de 1 à 4 % en poids.

7. Composition pharmaceutique contenant une(des) cyclosporine(s), selon une ou plusieurs des revendications précédentes, caractérisée en ce que l'on utilise comme huiles naturelles l'huile de soja et/ou l'huile de chardon et/ou des triglycérides à longueur moyenne de chaîne (par exemple l'huile de coprah) et/ou l'huile de germe de maïs et/ou l'huile de germe de blé et/ou l'huile de tournesol et/ou l'huile d'olive et/ou l'huile de poisson, et en ce que l'huile naturelle est de préférence dans une large mesure exempte de peroxydes.

8. Composition pharmaceutique contenant une(des) cyclosporine (s), selon une ou plusieurs des revendications précédentes, caractérisée en ce que la concentration des cyclosporines dans l'huile est comprise entre 0,2 et 7,0 % en poids, de préférence entre 1 et 5 % en poids.

9. Composition pharmaceutique contenant une (des) cyclosporsne(s), selon une ou: plusieurs des revendications précédentes, caractérisée en ce que l'huile naturelle est présente dans la composition pharmaceutique en une proportion de 5-40 %, de preférence en une proportion de 10-30 %, du poids total dans la composition pharmaceutique.

10. Composition pharmaceutique contenant une (des) cyclosporine(s), selon une ou plusieurs des revendications précédentes, caractérisée en ce qu'elle contient des sels alcalins d'acides gras libres saturés et/ou insaturés pharmaceutiquement acceptables, ayant de préférence de 6 à 26 atomes de carbone, les sels alcalins de l'acide palmitique et/ou l'acide palmitoléique et/ou l'acide linoléique et/ou l'acide linoléique, étant utilisés de préférence en tant que sels alcalins d'acides gras libres.

11. Composition pharmaceutique contenant une(des) cyclosporine(s), selon une ou plusieurs des revendications précédentes, caractérisée en ce que la teneur en sels alcalins d'acides gras libres va de 0,01 à 0,15 % en poids, de préférence de 0,02 à 0,06 % en poids.

12. Composition pharmaceutique contenant une(des) cyclosporine(s), selon une ou plusieurs des revendications précédentes, caractérisée en ce que la teneur en eau de la composition pharmaceutique va de 60 à 95 % en poids, de préférence de 70 à 90 % en poids.

13. Composition pharmaceutique contenant une (des) cyclosporine(s), selon une ou plusieurs des revendications précédentes, caractérisée en ce que la composition pharmaceutique contient en outre du glycérol en une quantité telle qu'elle est appropriée pour rendre isotonique la composition pharmaceutique.

14. Composition pharmaceutique contenant une (des) cyclosporine (s), selon une ou plusieurs des revendications précédentes, caractérisée en ce que les cyclosporines dissoutes dans les huiles naturelles sont incluses à l'intérieur de particules lipidiques constituées d'huiles naturelles, sont entourées d'une enveloppe constituée de 3-sn-phosphatidylcholine et/ou de phosphatidyléthanolamine, et sont émulsionnées dans de l'eau.

15. Procédé pour la préparation de la composition pharmaceutique contenant une (des) cyclosporine(s), selon une ou plusieurs des revendications précédentes, caractérisé en ce que les cyclosporines sont dissoutes dans des huiles naturelles et ensuite émulsionnées, d'une façon connue en soi, ayec de la 3-sn-phosphatidylcholine et/ou de la phosphatidyl-éthanolamine et de l'eau, et dans lequel sont ajoutés avant l'émulsification, ou dans lequel des acides gras des sels alcalins d'acides gras libres et/ou des sels alcalins d'acide gras sont contenus dans le mélange de cyclosporines, d'huiles naturelles, de 3-sn-phosphatidylcholine et/ou de phosphatidyléthanolamine et où on ajoute une solution alcaline avant l'émulsification.

16. Procédé selon la revendication 15, caractérisé en ce que les cyclosporines sont totalement dissoutes, avant l'émulsification, dans l'huile naturelle, et ensuite émulsionnées dans l'eau en une émulsion brute, à l'aide de 3-sn-phosphatidylcholines et de sels alcalins d'acides gras libres, et le mélange est ensuite homogénéisé en une émulsion, ayant de préférence une taille de particules inférieures à 4 µm, en particulier inférieure à 1,5 µm,

17. Procédé selon une ou plusieurs des revendications précédentes, caractérsé en ce que le mélange de cyclosporines, huiles naturelles, 3-sn-phosphatidylcholine et eau contient déjà 0,01-0,15 % d'acides gras naturels libres et/ou de leurs sels alcalins, et ce mélange est ajusté à un pH de 5-10, de préférence 7-9, au moyen d'une solution alcaline, avant l'émulsification.

18. Procédé selon une ou plusieurs des revendications précédentes, caractérisé en ce que les sels alcalins d'acides gras libres sont utilisés pour ajuster l'émulsion à un pH de 5-10, de préférence 7,0-9,0.

19. Procédé selon une ou plusieurs des revendications précédentes, caractérisé en ce que des solutions pour l'essentiel exemptes d'oxygène sont utilisées pour la préparation de la composition pharmaceutique.

20. Procédé selon une ou plusieurs des revendications précédentes, caractérisé en ce que l'on utilise des sels alcalins d'acides gras saturés et/ou insaturés pharmaceutiquement acceptables.

21. Procédé selon une ou plusieurs des revendications précédentes, caractérisé en ce que l'on utilise en outre du glycérol en une quantité qui est appropriée pour rendre isotonique la composition pharmaceutique.

22. Procédé selon une ou plusieurs des revendications précédentes, caractérisé en ce que l'on prépare d'abord une émulsion brute, par agitation énergique, et on effectue ensuite l'émulsification finale dans un homogenéisateur à haute pression, sous des pressions comprises entre 100 et 500 bars, l'homogénéisateur à haute pression comportant de préférence au moins trois pistons.

23. Procédé selon une ou plusieurs des revendications précédentes, caractérisé en ce que la stérilisation de la composition pharmaceutique est effectuée par stérilisation à la chaleur.

24. Utilisation de la composition pharmaceutique selon une ou plusieurs des reyendications précédentes, pour une injection intraveineuse et/ ou une perfusion.
